(19) Europäisches Patentamt / European Patent Office / Office européen des brevets

(11) **EP 2 818 157 A1**

(12) **EUROPEAN PATENT APPLICATION**
published in accordance with Art. 153(4) EPC

(43) Date of publication:
**31.12.2014 Bulletin 2015/01**

(51) Int Cl.:
*A61K 8/42* *(2006.01)*        *A61K 8/31* *(2006.01)*
*A61Q 19/00* *(2006.01)*

(21) Application number: **13751415.4**

(22) Date of filing: **22.01.2013**

(86) International application number:
**PCT/JP2013/051213**

(87) International publication number:
**WO 2013/125281 (29.08.2013 Gazette 2013/35)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **24.02.2012  JP 2012039372**
            **26.09.2012  JP 2012213109**

(71) Applicant: **FUJIFILM Corporation**
**Tokyo 106-8620 (JP)**

(72) Inventors:
- **KANAZAWA, Katsuhiko**
  **Ashigarakami-gun**
  **Kanagawa 258-8577 (JP)**
- **KITAOKA, Hiroyuki**
  **Ashigarakami-gun**
  **Kanagawa 258-8577 (JP)**
- **KUSUDA, Fumi**
  **Ashigarakami-gun**
  **Kanagawa 258-8577 (JP)**

(74) Representative: **Hoffmann Eitle**
**Patent- und Rechtsanwälte PartmbB**
**Arabellastraße 30**
**81925 München (DE)**

(54) **LYCOPENE-CONTAINING COMPOSITION**

(57)    Provided is a lycopene-containing composition containing lycopene and iodopropynyl butylcarbamate.

EP 2 818 157 A1

**Description**

Technical Field

The present invention relates to a lycopene-containing composition.

Background Art

Lycopene is an oil-soluble carotenoid dye that is distributed in plants. It has been reported in the studies conducted in recent years that lycopene exhibits useful activities such as an active oxygen scavenging action and a carcinogenesis suppressing action. Therefore, utilization of lycopene is expected in both preparations for internal use and preparations for external use, and various investigations have been conducted thereon (see, for example, Japanese Patent Application Laid-Open (JP-A) No. 2002-125619 and JP-A No. H11-35444).
Meanwhile, carotenoid dyes are known to be easily oxidized by light, heat or the like, and various methods for stabilizing carotenoid dyes have been investigated.
For example, Japanese Patent No. 4398246 discloses a carotenoid suspension exhibiting favorable emulsion stability, in which a carotenoid dye is combined with a predetermined amount of an emulsifier having an HLB value of from 12 to 18.
Furthermore, Japanese Patent No. 3921305 discloses a carotenoid aggregate having a particular form based on an appropriate mixing ratio between water and an organic solvent, which is used as a colorant having favorable light stability.
Compositions such as cosmetic products and food products include various antiseptic components in order to suppress the occurrence of microorganism from a hygienic viewpoint. A known example of such an antiseptic component is iodopropynyl butylcarbamate (see, for example, JP-ANos. H05-148127 and 2010-229081).

Citation List

Patent Document

    Patent Document 1: JP-ANo. 2002-125619
    Patent Document 2: JP-ANo. H11-35444
    Patent Document 3: Japanese Patent No. 4398246
    Patent Document 4: Japanese Patent No. 3921305
    Patent Document 5: JP-A No. H05-148127
    Patent Document 6: JP-A No. 2010-229081

SUMMARY OF INVENTION

Technical Problem

However, among carotenoids, lycopene is a compound which has particularly inferior stability, and it has become clear that, for example, lycopene becomes particularly unstable depending on the combination with particular antibiotic compounds that are generally used in view of the antiseptic performance of a formulation.
An object of the invention is to provide a lycopene-containing composition which has sufficient antiseptic performance and in which lycopene can be stably incorporated. Solution to Problem
The invention relates to the following.

    [1] A lycopene-containing composition containing lycopene and iodopropynyl butylcarbamate.
    [2] The lycopene-containing composition described in the item [1], wherein:

        the composition further contains at least one antibiotic compound having a minimum inhibitory concentration (MIC) against *Escherichia coli* of 10% by mass or less, and
        the antibiotic compound includes one selected from the group consisting of phenoxyethanol, an ether compound having from 6 to 11 carbon atoms in total formed between glycerin and an aliphatic alcohol, and an aliphatic diol compound having a carbon chain length of from 3 to 10.

[3] The lycopene-containing composition described in the item [2], wherein a sum of an effective coefficient of iodopropynyl butylcarbamate represented by the following Formula (I), and a total of effective coefficients of the antibiotic compound represented by the following Formula (II) is from 1.5 to 4.5:

Effective coefficient I = [Content (mass%) of iodopropynyl butylcarbamate] / [minimum inhibitory concentration (MIC) (mass%) of iodopropynyl butylcarbamate against *Escherichia coli*]    (I)

Effective coefficient II = [Content (mass%) of antibiotic compound] / [minimum inhibitory concentration (MIC) (mass%) of antibiotic compound against *Escherichia coli*] (II).

[4] The lycopene-containing composition described in the item [2] or [3], wherein the ether compound formed between glycerin and an aliphatic alcohol is ethylhexylglycerin.

[5] The composition described in any one of the items [2] to [4], wherein the aliphatic diol compound is at least one selected from pentylene glycol, hexylene glycol, heptanediol, or octanediol.

[6] The lycopene-containing composition described in any one of the items [1] to [5], further containing ascorbic acid.

[7] The lycopene-containing composition described in the item [6], wherein a content of the ascorbic acid is from 0.01% by mass to 0.5% by mass with respect to a total mass of the composition.

[8] The lycopene-containing composition described in any one of the items [1] to [7], wherein a content of the iodopropynyl butylcarbamate is from 0.0001% by mass to 0.5% by mass with respect to a total mass of the composition.

[9] The lycopene-containing composition described in any one of the items [1] to [8], wherein the lycopene-containing composition is an oil-in-water type emulsified composition containing lycopene as one of oil phase components, and a content ratio of aqueous phase components is 70% by mass or more with respect to a total amount of the composition.

[10] An external preparation for skin, including the lycopene-containing composition described in any one of the items [1] to [9].

Advantageous Effects of Invention

According to the invention, a composition containing lycopene, which has sufficient antiseptic performance and in which lycopene can be stably incorporated, can be provided.

DESCRIPTION OF EMBODIMENTS

The lycopene-containing composition of the invention is a lycopene-containing composition containing lycopene and iodopropynyl butylcarbamate.

According to the findings of the inventors of the invention, it was proved that when lycopene is combined with a benzoic acid ester compound that is usually used as a main antiseptic agent in an amount that is usually used, stability of lycopene is markedly impaired.

In this invention, when iodopropynyl butylcarbamate is used for such particularly unstable lycopene, a lycopene-containing composition having satisfactory antiseptic performance and having lycopene stably incorporated therein can be provided.

Hereinafter, the invention will be explained.

The term "stability over time" as used in the invention means that after a lycopene-containing composition is prepared, stability of the composition is sustained without being impaired over time.

The numerical value range indicated using the expression "A to B" in the present specification represents a range that includes the values A and B as the minimum value and the maximum value, respectively.

In this invention, the term "aqueous phase" is used as a term opposite to the "oil phase", irrespective of the kind of the solvent.

The term "process" as used in the present specification includes not only independent processes, but even in a case in which a process cannot be clearly distinguished from another process, the relevant process is intended to be included in the present term as long as a predetermined purpose of the present process is achieved.

In this invention, regarding the amount of each of the components in the composition, when there exist plural substances corresponding to each component in the composition, unless particularly stated otherwise, the amount of each component is intended to mean a total amount of the corresponding plural substances.

<Lycopene>

Lycopene according to the invention is a carotenoid represented by chemical formula: $C_{40}H_{56}$ (molecular weight: 536.87), and is a red dye which belongs to carotenes, one kind of carotenoids, and exhibits the maximum absorption at 474 nm (acetone).

Lycopene includes isomers of cis-form and trans-form, both having conjugated double bonds at the center of the molecule, and examples include all trans-form, 9-cis-form, and 13-cis-form. However, in this invention, any one of these may be used.

Lycopene may be included in the composition of the invention as an oil or paste containing lycopene that has been separated or extracted from a natural product containing lycopene.

In nature, lycopene is contained in tomato, persimmon, watermelon, and pink grapefruit. The oil containing lycopene as described above may be an oil separated or extracted from such a natural product. Regarding the form of manufactured product, four kinds such as an oil type, an emulsion liquid type, a paste type, and a powder type are known.

Also, lycopene that is used in the invention may be an extract of the natural products described above, or a product obtained by further purifying this extract as appropriate and as necessary, or may also be a synthetic product.

One particularly preferred form of lycopene according to the invention is an oil-soluble extract extracted from tomato pulp. The oil-soluble extract extracted from tomato pulp is particularly preferred from the viewpoints of stability in a composition containing the relevant oil-soluble extract, product quality, and productivity.

Here, an oil-soluble extract extracted from tomato pulp means an extract extracted using an oily solvent, from a pulp-like solid obtainable by centrifuging a crushed product obtained by crushing tomatoes.

Regarding lycopene, which is an oil-soluble extract, a tomato extract that is widely marketed as an oil or paste containing lycopene can be used, and examples include Lyc-O-Mato 15% and Lyc-O-Mato 6% commercially available by Sunbright Co., Ltd.; and LYCOPENE 18 commercially available from Kyowa Hakko Kogyo Co., Ltd.

A content of lycopene in the composition of the invention may vary depending on the formulation, but generally, a content is preferably from 0.00001% by mass to 5% by mass, more preferably from 0.00005% by mass to 0.5% by mass, and still more preferably from 0.0001% by mass to 0.05% by mass, with respect to a total mass of the composition.

<Iodopropynyl Butylcarbamate>

Iodopropynyl butylcarbamate according to the invention is an essential component for exhibiting favorable antiseptic performance (antibiotic performance) without impairing the stability of lycopene.

A content of iodopropynyl butylcarbamate is not particularly limited as long as the antiseptic effect can be exhibited without impairing the stability of lycopene, but from the viewpoint of antiseptic performance, a content is preferably from 0.0001% by mass to 0.5% by mass, and more preferably from 0.005% by mass to 0.05% by mass, with respect to a total mass of the composition. When a content is 0.0001% by mass or more, the composition tends to exhibit an antiseptic effect favorably, and when a content is 0.5% by mass or less, the composition tends not to have excessively strong irritancy to the skin.

Iodopropynyl butylcarbamate is available as commercially marketed products, and examples include GLYCACIL 2000, L, and S (manufactured by Lonza Group AG).

The minimum inhibitory concentration (MIC) of iodopropynyl butylcarbamate against *E. coli* is 0.01% by mass.

<Antibiotic Compound>

The lycopene-containing composition of the invention may contain a compound having an antibiotic (antiseptic) effect, in addition to iodopropynyl butylcarbamate. Thereby, the antiseptic performance of the composition can be maintained favorably.

Examples of a compound that can be added, from which an antiseptic effect can be expected in the invention, include antibiotic compounds having a minimum inhibitory concentration (MIC) against *E. coli* of from 0.001% by mass to 10% by mass. Unless particularly stated otherwise, the "antibiotic compound" according to the invention does not include iodopropynyl butylcarbamate.

The minimum inhibitory concentration (MIC) means the minimum amount (mass%) of an antiseptic agent required to inhibit proliferation of a particular bacterium. A smaller MIC value means a stronger antiseptic effect of the compound, and on the contrary, a larger value thereof implies that the compound needs to be added in a large amount in order to satisfy the antiseptic effect to a certain extent.

The bacterium that is subject to the MIC will be defined as *Escherichia coli.* The minimum inhibitory concentration (MIC) can be determined by the following method. In this invention, unless particularly stated otherwise, "MIC" means the MIC against *E. coli* that can be determined by the following method.

MIC can be determined by the "Microbial limit test methods" among the general methods disclosed in "Japanese Pharmacopeia, 15th Revision (2006)". Examples of the microbial limit test methods include a membrane filtration method, an agar plate method, an agar plate surface smear method, and a liquid medium serial dilution method. For the MIC according to the invention, a value obtained by a liquid medium serial dilution method is used.

Examples of the antibiotic compound having a MIC value of 10% by mass or less include methyl para-oxybenzoate (MIC: 0.2% by mass), ethyl para-oxybenzoate (MIC: 0.1% by mass), propyl para-oxybenzoate (MIC: 0.05% by mass), pentylene glycol (MIC: 2.65% by mass), phenoxyethanol (MIC: 0.4% by mass), ethylhexylglycerin (MIC: 0.2% by mass), disodium edetate (MIC: 0.02% by mass), 1,3-butylene glycol (MIC: 10% by mass), salicylic acid (MIC: 0.06% by mass), dehydroacetic acid (MIC: 0.008% by mass), benzoic acid (MIC: 0.1% by mass), sorbic acid (MIC: 0.2% by mass), propylene glycol (MIC: 10% by mass), hinokitiol (MIC: 0.002% by mass), and isopropylmethylphenol (MIC: 0.015% by mass).

Among them, from the viewpoint of suppression of the decomposition of lycopene, an antibiotic compound having a MIC value of 3% by mass or less is preferred.

From the viewpoint of achieving a balance between the stability of lycopene and the antiseptic performance, the antibiotic compound is preferably one selected from the group consisting of phenoxyethanol, an ether compound having from 6 to 11 carbon atoms in total formed between glycerin and an aliphatic alcohol, and an aliphatic diol compound having a carbon chain length of from 3 to 10.

In regard to the ether compound formed between glycerin and an aliphatic alcohol, the aliphatic alcohol that can form an ether bond with glycerin may be straight-chained or branched. The ether compound is preferably, for example, ethylhexylglycerin.

In regard to the aliphatic diol compound having a carbon chain length of from 3 to 10, the carbon chain length means the number of consequent carbon atoms that constitute an aliphatic chain to which two hydroxyl groups are bonded. Examples of the aliphatic diol having a carbon chain length of from 3 to 10 include butanediol, pentylene glycol, hexylene glycol, heptanediol, octanediol, and decanediol. The positions of substituent of hydroxyl groups may be any positions, and the carbon chain may be straight-chained or branched. From the viewpoint of the strength of antibiotic performance, the aliphatic diol compound is preferably at least one selected from the group consisting of pentylene glycol, hexylene glycol, heptanediol, or octanediol.

<Effective Coefficient as Composition>

From the viewpoint of the overall antiseptic performance of the lycopene-containing composition according to the invention, it is preferable that the sum of an effective coefficient of iodopropynyl butylcarbamate represented by the following Formula (I) and a total of effective coefficients of the antibiotic compound represented by the following Formula (II) is from 1.5 to 4.5:

Effective coefficient I = [Content (mass%) of iodopropynyl butylcarbamate] / [minimum inhibitory concentration (MIC) (mass%) of iodopropynyl butylcarbamate against *Escherichia coli*]     (I)

Effective coefficient II = [Content (mass%) of antibiotic compound] / [minimum inhibitory concentration (MIC) (mass%) of antibiotic compound against *Escherichia coli*]     (II).

Thereby, lycopene decomposition can be suppressed while the overall antiseptic performance of the composition is reliably secured.

When two or more antibiotic compounds described above are present in the composition, the respective effective coefficients II of the antibiotic compounds is determined, and it is preferable that a sum of a total of the effective coefficients II of the antibiotic compounds and a total of the effective coefficient I of iodopropynyl carbamate falls within the range described above.

The effective coefficients represented by Formula (I) and Formula (II) are values obtained by dividing the contents (mass%) of the subject iodopropynyl carbamate and antibiotic compound in the composition, by the respective values of MIC. A larger value of the effective coefficient means a higher antiseptic effect.

From the viewpoints of favorable overall antiseptic performance of the composition and reliable suppression of the decomposition of lycopene, a sum of an effective coefficient I of iodopropynyl carbamate and a total of the effective coefficients II of the antibiotic compounds in the composition is preferably from 1.5 to 4.5, and more preferably from 1.7 to 3.5, from the viewpoints of the antiseptic performance for the composition and the suppression of decomposition of lycopene.

In the lycopene-containing composition of the invention, other components in addition to lycopene, iodopropynyl carbamate and the antibiotic compound, can be appropriately selected and incorporated in accordance with the form, purpose and the like of the composition. Suitable examples of the other components include an antioxidant, a functional oily component, a surfactant, a phospholipid, and other additive components.

(Antioxidant)

The lycopene-containing composition according to the invention preferably further includes an antioxidant, from the viewpoint of enhancing stability of lycopene.

Examples of the antioxidant include an ascorbic acid compound, dibutylhydroxytoluene, and a tocopherol; however, from the viewpoint of noticeably enhancing the stability of lycopene, an ascorbic acid compound is particularly preferred. Examples of the ascorbic acid compound include ascorbic acid, sodium ascorbate, magnesium ascorbate, magnesium ascorbate sulfate, sodium ascorbate sulfate, magnesium ascorbate phosphate, sodium ascorbate phosphate, ascorbic acid glucoside, and ascorbic acid palmitate, and it is preferable that the lycopene-containing composition include at least one selected from these ascorbic acid compounds. Among them, when the lycopene-containing composition includes ascorbic acid, it is particularly preferable because decomposition of lycopene can be markedly suppressed.

An amount of incorporation of the ascorbic acid in the lycopene-containing composition of the invention may be from 0.01% by mass to 0.5% by mass, and preferably from 0.1% by mass to 0.5% by mass, with respect to a total amount of the composition. When an amount of incorporation of the ascorbic acid is 0.01% by mass or more, the effect of suppressing the decomposition of lycopene is more effectively exhibited. Also, when an amount of incorporation of the ascorbic acid is 0.5% by mass or less, coloration or odor generation caused by change over time in ascorbic acid can be favorably suppressed.

(Functional Oily Component)

The functional oily component is not particularly limited as long as it is an oil-soluble component that does not dissolve in an aqueous medium but dissolves in an oily medium, and any compound having the property or functionality that complies with the purpose can be appropriately selected and used. Examples of the other oil component include components that are usually used as an ultraviolet absorber, an anti-inflammatory agent, a moisturizing agent, a hair protective agent, a skin-lightening agent, an anti-blemish agent, a cell activator, an emollient agent, a keratolytic agent, an antistatic agent, oil-soluble vitamins, a metabolic syndrome ameliorant, an antihypertensive agent, and a sedative.

Here, a functional oily component means an oily component which can be expected to exhibit a desired physiological action in a living body when the component is available in a living organism.

Examples of the functional oily component include ceramides including ceramide and ceramide analogues, such as naturally occurring ceramides, and sugar-modified ceramides such as sphingoglycolipids; oils and fats such as olive oil, camellia oil, macadamia nut oil, castor oil, and coconut oil; hydrocarbons such as liquid paraffin, paraffin, petrolatum, ceresin, microcrystalline wax, and squalane; waxes such as carnauba wax, candelilla wax, jojoba oil, beeswax, and lanolin; esters such as isopropyl myristate, 2-octyldodecyl myristate, cetyl 2-ethylhexanoate, and diisostearyl malate; fatty acids such as palmitic acid, stearic acid, and isostearic acid; higher alcohols such as cetyl alcohol, stearyl alcohol, isostearyl alcohol, and 2-octyldodecanol; silicone oils such as methylpolysiloxane and methylphenylpolysiloxane; fatty acid esters of glycerin; oil-soluble vitamins such as vitamin E (tocopherol), vitamin A, and vitamin D; as well as polymers, oil-soluble dyes such as carotenoids other than lycopene (astaxanthin and the like), and oil-soluble proteins. Furthermore, various plant-derived oils and animal-derived oils, which are mixtures of those compounds, are also included.

(Surfactant)

The lycopene-containing composition of the invention may contain one kind or two or more kinds of surfactants.

The surfactant is a component which can function as an emulsifier in a case in which the lycopene-containing composition of the invention is configured as an emulsified composition, or the like.

The surfactant that can be applied to the invention may be any one of an anionic surfactant, a cationic surfactant, an amphoteric surfactant, and a nonionic surfactant (hereinafter, also referred to as "nonionic surfactant").

Furthermore, from the viewpoint of the emulsifying power, the surfactant according to the invention preferably has an HLB value of 10 or more, and more preferably 12 or more. If the HLB value is too low, the emulsifying power may be insufficient. From the viewpoint of a defoaming effect, a surfactant having an HLB value of 5 or more but less than 10 may also be used in combination.

Here, the HLB means hydrophilicity-hydrophobicity balance that is conventionally used in the field of surfactants, and can be determined using a calculation formula that is usually used, for example, Kawakami's formula. Kawakami's formula will be shown below.

$$HLB = 7 + 11.7\log(Mw/Mo)$$

Here, Mw represents the molecular weight of a hydrophilic group, and Mo represents the molecular weight of a hydrophobic group.

The values of HLB described in catalogues and the like may also be used.

Also, as is understood from the above formula, an emulsifier having an arbitrary HLB value can be obtained by utilizing additivity of the HLB.

Among the surfactants, nonionic surfactants are preferred since these are less irritating and have less influence on the environment. Examples of the nonionic surfactants include fatty acid esters of monosaccharides or polysaccharides, polyglycerin fatty acid esters, organic acid monoglyceride, propylene glycol fatty acid esters, polyglycerin-condensed ricinoleic acid ester, sorbitan fatty acid esters, and polyoxyethylene sorbitan fatty acid esters. Among these, at least one selected from fatty acid esters of monosaccharides or polysaccharides, or polyglycerin fatty acid esters can be suitably used.

Examples of the monosaccharides or polysaccharides that constitute the fatty acid esters of monosaccharides or polysaccharides include glucose and sucrose.

Regarding the fatty acid esters of monosaccharides or polysaccharides, an ester form between a monosaccharide or a polysaccharide and a straight-chained or branched fatty acid having from 6 to 22 carbon atoms is more preferred. From the viewpoint that a highly stable lycopene-containing composition can be produced, sucrose myristic acid ester, sucrose palmitic acid ester, sucrose stearic acid ester, and sucrose oleic acid ester are still more preferred, and sucrose stearic acid ester is most preferred.

Regarding the polyglycerin fatty acid esters, an ester form between a polyglycerin having a degree of polymerization of 2 or higher and a straight-chained or branched fatty acid having from 6 to 22 carbon atoms is more preferred.

Still more preferred examples of the polyglycerin fatty acid esters include, from the viewpoint of producing an external preparation for skin having high stability, polyglyceryl-6 laurate, polyglyceryl-6 myristate, polyglyceryl-6 stearate, polyglyceryl-6 oleate, polyglyceryl-10 laurate, polyglyeryl-10 myristate, polyglyceryl-10 stearate, decaglyceryl monooleate, and polyglyceryl-10 oleate. Also, decaglyceryl monooleate and decaglyceryl-10 oleate are most preferred.

(Phospholipids)

The lycopene-containing composition of the invention may also include a phospholipid such as lecithin. The phospholipid is a component which can function as an emulsifier in a case in which the external preparation for skin of the invention is configured as an emulsified composition, or the like.

The phospholipid that can be used in the invention is a compound containing a glycerin skeleton, and a fatty acid residue and a phosphoric acid residue as essential constituent components, with a base, a polyhydric alcohol or the like being bonded thereto, and this is also referred to as lecithin. Since a phospholipid has a hydrophilic group and a hydrophobic group in the molecule, phospholipids have been hitherto used widely as emulsifiers in the fields of food, pharmaceutics, and cosmetics.

From an industrial aspect, a lecithin having a purity of 60% or higher is used as lecithin, and this can also be used in this invention. However, from the viewpoints of forming oil droplets having fine particle sizes, and obtaining stability of the functional oily component, a lecithin generally referred to as high purity lecithin is preferred, and this has a lecithin purity of 80% or higher, and more preferably 90% or higher.

Examples of the phospholipids include various conventionally known phospholipids that are extracted and separated from living bodies of plants, animals, and microorganisms.

Specific examples of such phospholipids include various lecithins derived from plants such as soybean, corn, peanut, rapeseed, and barley; animals such as egg yolk and beef; microorganisms such as E. coli; and the like. Among these, egg yolk-derived lecithin or soybean-derived lecithin is preferred, and soybean-derived lecithin is more preferred.

Examples of such lecithins include, as expressed in compound names, glycerolecithins such as phosphatidic acid, phosphatidylglycerin, phosphatidylinositol, phosphatidylethanolamine, phosphatidylmethylethanolamine, phosphatidylcholine, phosphatidylserine, bisphosphatidic acid, diphosphatidylglycerin (cardiolipin), phosphatidylethanolamine, phosphatidylinositol, and lysophosphatidylcholine; and sphingolecithins such as sphingomyelin.

According to the invention, hydrogenated lecitihin, enzymatically degraded lecithin, enzymatically degraded hydrogenated lecithin, hydroxylecithin, and the like can also be used in addition to the high purity lecithin. These lecithins that can be used in the invention can be used singly or in mixture of plural kinds thereof.

(Other Additive Components)

In addition to the components described above, additive components that are conventionally used in the fields of food, cosmetics and the like may also be incorporated into the lycopene-containing composition of the invention as appropriate, according to the form of the composition.

Other additive components can be incorporated into the lycopene-containing composition of the invention as oil-soluble or water-soluble additive components, in accordance with the characteristics of the components.

Examples of the other additive components include polyhydric alcohols such as glycerin; monosaccharides or polysaccharides, such as kappa-carrageenan, locust bean gum, guar gum, hydroxypropyl guar gum, xanthan gum, karaya gum, tamarind seed polysaccharide, gum arabic, tragacanth gum, hyaluronic acid, sodium hyaluronate, sodium chondroitin sulfate, and dextrin; sugar alcohols such as sorbitol, mannitol, maltitol, lactose, maltotriitol, and xylyrtol; inorganic salts such as sodium chloride and sodium sulfate; proteins having molecular weights of more than 5000, such as casein, albumin, methylated collagen, hydrolyzed collagen, water-soluble collagen, and gelatin; amino acids and derivatives thereof, such as glycine, alanine, valine, leucin, isoleucin, serine, threonine, aspartic acid, glutamic acid, cysteine, methionine, lysine, hydroxylysine, arginine, histidine, phenylalanine, tyrosine, tryptophan, proline, hydroxyproline, and acetylhydroxyproline; synthetic polymers such as carboxyvinyl polymers, sodium polyacrylate, polyvinyl alcohol, polyethylene glycol, an ethylene oxide-propylene oxide block copolymer; water-soluble cellulose derivatives such as hydroxyethyl cellulose and methyl cellulose; flavonoids (catechin, anthocyanin, flavone, isoflavone, flavan, flavanone, rutin), ascorbic acid or derivatives thereof (ascorbic acid, sodium ascorbate, potassium ascorbate, calcium ascorbate, L-ascorbic acid phosphoric acid ester, magnesium ascorbyl phosphate, sodium ascorbyl phosphate, ascorbyl sulfate, disodium ascorbyl sulfate, ascorbyl 2-glucoside, and the like), tocotrienol and derivatives thereof, phenolic acids (chlorogenic acid, ellagic acid, gallic acid, and propyl gallate), lignans, curcumins, coumarins, and hydroxystilbene including pterostilbene. These components may be included as, for example, functional components, excipients, viscosity adjusting agents, and radical scavengers, based on their functions.

In addition to that, according to the invention, other additives that are usually used for the applications such as, for example, various efficacious components, pH adjusting agents, pH buffering agents, ultraviolet absorbers, antiseptic agents other than the aforementioned antibiotic compounds, fragrances, and colorants, can be used in combination.

There are no particular limitations on the form of the lycopene-containing composition of the invention, and the composition may be in the form of a composition including a pharmaceutically acceptable carrier, and other optional components used as necessary, in addition to active ingredients. Examples of the form of the composition include an oil composition and an emulsified composition. The emulsified composition is preferably an oil-in-water type emulsified composition containing lycopene as one of oil phase components.

[Method for Producing Lycopene-Containing Composition]

The method for producing the lycopene-containing composition of the invention is not particularly limited, and the lycopene-containing composition can be produced according to a known method. For example, in the case of obtaining a lycopene-containing composition in the form of an emulsified composition, preferred is a production method including the steps of: a) dissolving a nonionic surfactant in an aqueous medium (water or the like), and thereby obtaining an aqueous phase; b) mixing and dissolving oily components including lycopene, and thereby obtaining an oil phase composition; and c) mixing this aqueous phase composition and this oil phase composition with stirring to perform emulsifying dispersion, and obtaining a lycopene-containing composition.

There are no particular limitations on the components that are incorporated into the oil phase composition and the aqueous phase composition in the case of producing a lycopene-containing composition by the production method described above, but from the viewpoint of obtaining a highly stable lycopene-containing composition, it is preferable to incorporate components such as surfactants described above, and it is preferable to incorporate any one or plural kinds of i) fatty acid esters of monosaccharides or polysaccharides, ii) polyglycerin fatty acid esters, and iii) phospholipids.

A ratio (by mass) between the oil phase and the aqueous phase on the occasion of the emulsifying dispersion described above is not particularly limited, but a ratio of oil phase/aqueous phase (mass%) is preferably 0.1/99.9 to 50/50, more preferably 0.5/99.5 to 30/70, and still more preferably 1/99 to 20/80.

When a ratio of oil phase/aqueous phase is adjusted to 0.1/99.9 or more, since a content of active ingredients is not excessively lowered, there is a tendency that no problem occurs in the practical use of the lycopene-containing composition, and thus it is preferable. Also, when a ratio of oil phase/aqueous phase is adjusted to 50/50 or less, the surfactant concentration is not diluted, and there is a tendency that emulsification stability of the lycopene-containing composition is not deteriorated, and thus it is preferable.

It is particularly preferable that the lycopene-containing composition be an oil-in-water type emulsified composition in which a content ratio of the aqueous phase components is 70% by mass or more with respect to a total mass of the composition, from the viewpoint that the effects of the invention may be exhibited more favorably.

Emulsifying dispersion may be achieved by performing a single-step emulsification operation, but it is preferable to perform an emulsification operation of two or more steps from the viewpoint of obtaining uniform and fine particles.

Specifically, it is particularly preferable to use two or more kinds of emulsifying apparatuses in combination, by utilizing a method of performing emulsification through a high pressure homogenizer, an ultrasonic dispersing machine or the like, in addition to a single-step emulsification operation of performing emulsification using a conventional emulsifying apparatus utilizing a shearing action (for example, stirring by a stirrer or an impeller, a homomixer, or a continuous flow type shearing apparatus). When a high pressure homogenizer is used, the emulsion can be prepared into liquid droplets of more uniform fine particles. The operation may also be carried out several times for the purpose of making liquid droplets having a more uniform particle size.

Examples of methods known to be useful for micronizing an emulsion include surface chemical emulsification methods such as a PIT emulsification method and a gel emulsification method. These methods have an advantage of consuming less energy, and are appropriately used in the case of finely emulsifying a material that is prone to be deteriorated by heat.

Furthermore, as an emulsifying method that is used for general purpose, a method of using a mechanical force, that is, a method of splitting oil droplets by applying a strong shear force from an external source is applied. The most generally used mechanical force is a high speed, high shear stirrer. Examples of such a stirrer that is commercially available include stirrers referred to as a homomixer, a disper mixer, and an ultramixer.

There is also available a high pressure homogenizer as a special mechanical emulsifying apparatus useful for micronization, and various apparatuses are commercially available. Since a high pressure homogenizer can provide a large shear force compared with a stirring system, even if the amount of the emulsifier is relatively small, micronization can be achieved.

High pressure homogenizers are roughly divided into chamber type high pressure homogenizers having a fixed throttle part, and homogenizing valve type high pressure homogenizers of a type capable of controlling the opening of a throttle.

Examples of the chamber type high pressure homogenizers include a MICROFLUIDIZER (manufactured by Microfluidics Corp.), a NANOMIZER (manufactured by Yoshida Kikai Co., Ltd.), and an ULTIMIZER (manufactured by Sugino Machine, Ltd.).

Examples of the homogenizing valve type high pressure homogenizers include a Gaulin type Homogenizer (manufactured by APV Corp.), a Lanier type homogenizer (manufactured by Lanier Co., Ltd.), a high pressure homogenizer (manufactured by GEA Niro Soavi S.p.A.), a homogenizer (manufactured by Sanwa Machinery Trading Co., Ltd.), a high pressure homogenizer (manufactured by Izumi Food Machinery Co., Ltd.), and an ultrahigh pressure homogenizer (manufactured by IKA Group).

Regarding a dispersing apparatus having relatively high energy efficiency, there is available an ultrasonic homogenizer as an emulsifying apparatus having a simple structure. Examples of the high power output ultrasonic homogenizer capable of production include ULTRASONIC HOMOGENIZER US-600, ULTRASONIC HOMOGENIZER US-1200T, ULTRASONIC HOMOGENIZER RUS-1200T, and ULTRASONIC HOMOGENIZER MUS-1200T (all manufactured by Nissei Corp.); and ULTRASONIC PROCESSOR UIP2000, ULTRASONIC PROCESSOR UIP-4000, ULTRASONIC PROCESSOR UIP-8000, and ULTRASONIC PROCESSOR UIP-16000 (all manufactured by Hielscher Ultrasonics GmbH). These high power output ultrasonic irradiation apparatuses are used at a frequency of 25 kHz or less, and preferably from 15 kHz to 20 kHz.

Regarding another known emulsifying means, methods of using a static mixer, a microchannel, a micromixer, or a membrane emulsifying apparatus, which do not have a stirring unit from the outside and require only a low level of energy, are also useful.

There are no particular limitations on the temperature conditions at the time of performing emulsifying dispersion according to the invention; however, from the viewpoint of stability of the functional oily component, the temperature is preferably from 10°C to 100°C, and an appropriately preferred range can be selected depending on the melting point of the handled functional oily component, or the like.

Also, in the case of using a high pressure homogenizer in the invention, it is preferable to handle the process at a pressure of preferably 50 MPa or higher, more preferably from 50 MPa to 280 MPa, and still more preferably from 100 MPa to 280 MPa.

The emulsion liquid which is an emulsifying dispersed composition, is preferably cooled through a certain cooler within 30 seconds, and preferably within 3 seconds, immediately after the passage through the chamber, from the viewpoint of retaining the particle size of the dispersed particles.

<Applications>

Since the lycopene-containing composition of the invention has favorable antiseptic performance and favorable stability of lycopene, the composition can be applied to applications such as cosmetic applications and transdermal pharmaceutical applications, and it is particularly preferable that the lycopene-containing composition be applied as an external preparation for skin. That is, the external preparation for skin of the invention includes a lycopene-containing composition, and may be composed of a lycopene-containing composition alone.

Examples of the external preparation for skin of the invention include, but are not limited to, skin care cosmetic products (skin toner, essence, emulsion, cream, and the like), lipstick, ultraviolet-screening cosmetic products, and makeup cosmetics materials.

When the external preparation for skin of the invention is used particularly in aqueous products such as skin toner, essence, emulsion, cream, pack mask, pack, hair-washing cosmetics, fragrance cosmetics, liquid body washes, UV care cosmetics, deodorant cosmetics, and oral care cosmetics (in the case of cosmetic products), products having a feeling of transparency may be obtained, and the occurrence of inappropriate phenomena such as precipitation, sedimentation or neck ring of insoluble matters under severe conditions such as long-term storage or a sterilization treatment can be suppressed.

EXAMPLES

Hereinafter, the invention will be described in detail by way of Examples, but the invention is not intended to be limited thereto.

[Example 1 to Example 7, Comparative Example 1 to Comparative Example 5, and Reference Example 1]

«Production of Lycopene-Containing Emulsified Composition»

[Oil Phase Composition]
• Tomato oleoresin                                                1.14 g
(manufactured by Sunbright Co., Ltd., Lyc-O-Mato 15% (containing 15% by mass of lycopene))
• Mixed tocopherol                                               1.0 g
(manufactured by Riken Vitamin Co., Ltd., RIKEN E OIL 800)
• Medium-chain fatty acid glycerides                             12.8 g
(manufactured by Kao Corp., COCONARD MT)
• Diglycerin monostearate                                        0.5 g
(NIKKOL DGMS manufactured by Nikko Chemicals Co., Ltd.)

[Aqueous Phase Composition]

• Decaglyceryl oleate-10                                         8.0 g
(HLB = 12.0, manufactured by Nikko Chemicals Co., Ltd., Decaglyn 1-OV)
• Glycerin                                                       45.0 g
• Purified water                                                 Balance to make up 100 g

The various components used in the aqueous phase composition were weighed in a container according to the composition described above, and the components were heated and mixed while stirred in a constant temperature tank at 70°C. It was checked whether the mixture was thoroughly mixed, the mixture was maintained at 70°C, and thus an aqueous phase composition was obtained.

The various components used in the oil phase composition were weighed in a container according to the composition described above, and the components were heated and mixed for 5 minutes while stirred on a hot plate at 150°C. It was checked whether the mixture was thoroughly mixed, and thus an oil phase composition was obtained.

The aqueous phase composition thus obtained was added to the oil phase composition, and the mixture was stirred and mixed, and was dispersed for a predetermined time using an ultrasonic homogenizer. Thus, a crude dispersion liquid was obtained.

Thereafter, the crude dispersion thus obtained was further subjected to high pressure emulsification at 200 MPa using an ultrahigh pressure emulsifying apparatus (ULTIMIZER, manufactured by Sugino Machine Co., Ltd.), and thus a lycopene-containing composition (content ratio of lycopene: 0.17% by mass) was prepared.

The lycopene-containing emulsified composition thus obtained was diluted to 1% by mass with MilliQ water, and the particle size of the dispersed particles was measured using a particle size analyzer FPAR-1000 (Otsuka Electronics Co., Ltd.). The particle size was 52 nm (medium diameter (d = 50)).

<Production of Cosmetic Preparations>

Cosmetic preparations having the compositions indicated in the following Table 1 or Table 2 were produced by conventional methods, using the lycopene-containing emulsified composition obtained as described above. The numerical values explaining the compositions in Table 1 and Table 2 represent "mass%".

In Table 1, for hinokitiol, a product manufactured by Wako Pure Chemical Industries, Ltd. was used.

In Table 2, for the tomato extract, Lyc-O-Mato 6% (content ratio of lycopene: 0.1% by mass, manufactured by Sunbright Co., Ltd.) diluted with glycerin was used.

In Table 2, for the krill extract, ASTAX ST (manufactured by Itano Refrigerated Food Co., Ltd.) was used.

In Table 1, for the astaxanthin-containing emulsified composition, a composition prepared as follows was used.

«Production of Astaxanthin-Containing Emulsified Composition»

The components described below were dissolved for one hour while heated at 70°C, and thus an aqueous phase composition A was obtained.

| | |
|---|---|
| • Sucrose stearic acid ester (HLB = 16) | 33.0 g |
| • Decaglyceryl monooleate (HLB = 12) | 67.0 g |
| • Glycerin | 450.0 g |
| • Pure water | 300.0 g |

The components described below were dissolved for one hour while heated at 70°C, and thus an oil phase composition A was obtained.

| | |
|---|---|
| • Haematococcus algae extract | 15.0 g |
| (content ratio of astaxanthins: 20% by mass, product name: ASTAT S, Takeda Shiki Co., Ltd.) | |
| • Mixed tocopherol | 32.0 g |
| (manufactured by Riken Vitamin Co., Ltd., RIKEN E OIL 800) | |
| • Medium-chain fatty acid glycerides | 93.0 g |
| (manufactured by Kao Corp., COCONARD MT) | |
| • Lecithin | 10.0 g |
| (manufactured by Riken Vitamin Co., Ltd., LECION P, soybean-derived) | |

The aqueous phase composition A obtained as described above was stirred (10,000 rpm) with a homogenizer (model name: HP93, manufactured by SMT Corp.) while maintained at 70°C, and the oil phase composition A was added to the aqueous phase composition A. Thus, a preliminary emulsification product was obtained.

Subsequently, the preliminary emulsification product thus obtained was cooled to about 40°C, and was subjected to high pressure emulsification at a pressure of 200 MPa using an ULTIMIZER HJP-25005 (manufactured by Sugino Machine Co., Ltd.). Thereafter, the preliminary emulsification product was filtered through a microfilter having an average pore diameter of 1 μm, and thus an astaxanthin-containing emulsified composition (content ratio of astaxanthin: 0.3% by mass) was produced.

The astaxanthin emulsified composition thus obtained was diluted to 1% by mass with MilliQ water, and the particle size of the dispersed particles was measured using a particle size analyzer FPAR-1000 (Otsuka Electronics Co., Ltd.). The particle size was 58 nm (median diameter (d = 50)).

<Evaluation>

The stability over time of the cosmetic preparations obtained as described above was evaluated by measuring the residual ratio of lycopene. An evaluation of antiseptic performance was also carried out simultaneously.

<Stability Over Time>

External preparations for skin obtained in Examples 1 to 6, Comparative Examples 1 to 13, and Reference Examples 1 to 3 were respectively prepared in one glass bottle with a lid. The absorbances were measured immediately after production, and after storage over time for 2 weeks at 40°C or for 6 weeks at 40°C, under the following conditions. The change ratios (%) of absorbance were calculated by the following formula, using the measurement results thus obtained, and the external preparations for skin were evaluated according to the criteria from A to C described below, based on the residual ratios thus obtained. The results are presented in Table 1 and Table 2.

$$\text{Change ratio (\%)} = \text{Absorbance after the passage of time for 2 weeks or 3 weeks at } 40°C \text{ / absorbance immediately after production} \times 100$$

A: The lycopene residual ratio was 70% or more.
B: The lycopene residual ratio was 50% or more but less than 70%.
C: The lycopene residual ratio was less than 50%.

<Antiseptic Performance>

The antiseptic performance was evaluated according to "28. Preservatives-Effectiveness Tests" in the "Japanese Pharmacopoeia, 15th Revision" (2006).
Bacteria used were *Aspeigillus niger* ATCC16404 *(A. niger), Candida albicans* ATCC10231 (fungus), *Escherichia coli* ATCC8739 *(E. coli), Pseudomonas aeruginosa* ATCC9027 (*P. aeruginosa*), and *Staphylococcus aureus* ATCC6538 *(S. aureus).*
A preservatives-effectiveness test is a method for microbiologically evaluating the effectiveness of a preparation itself filled in a multiple-dose container, or of a preservative added to such a preparation. In the present test method, preservative power is evaluated by forcibly inoculating and mixing bacteria species to be tested into a preparation, and tracing the prosperity and decay of the test bacteria over time. The evaluation of antiseptic performance of the lycopene-containing composition was carried out as follows based on the state of bacteria, according to the procedure for Category I preparations in "28. Preservatives-Effectiveness Tests".

A: The cell density was at or below the detection limit within 2 weeks for all of the bacteria.
B: The cell density was at or below the detection limit within 4 weeks, or for the fungus, a bacteriostatic effect could be confirmed.
C: Even after 4 weeks, 103 or more living cells were recognized.

The results are presented in Table 1 and Table 2.

[Table 1]

| Composition | | Example 1 | Example 2 | Example 3 | Example 4 | Comparative Example 1 | Comparative Example 2 |
|---|---|---|---|---|---|---|---|
| Composition | Lycopene-containing emulsified composition | 0.20 | 0.20 | 0.20 | 0.20 | 0.20 | 0.20 |
| | Astaxanthin-containing emulsified composition | - | - | - | - | - | - |
| | Magnesium ascorbate, phosphate | 0.10 | 0.10 | 0.10 | 0.10 | 0.10 | 0.10 |
| | 1,3-BG | 2.00 | 2.00 | 2.00 | 2.00 | 2.00 | 2.00 |
| | Pentylene glycol | 2.00 | 2.00 | 2.00 | 2.00 | 2.00 | 2.00 |
| | Hydrogenated hardened castor oil | 0.12 | 0.12 | 0.12 | 0.12 | 0.12 | 2.12 |
| | Tocopherol | - | - | - | - | - | - |
| | Propylene glycol | 2.00 | 2.00 | 2.00 | 2.00 | 2.00 | 2.00 |
| | Iodopropynyl butylcarbamate | 0.008 | 0.002 | 0.001 | 0.001 | | |
| | Phenoxyethanol | | 0.25 | | | | |
| | Ethylhexylglycerin | | | 0.25 | | | |
| | Hinokitiol | | | | 0.002 | | |
| | Methlparaben | | | | | | |
| | Ethylparaben | | | | | | |
| | Propylparaben | | | | | | |
| | Isopropylmethylphenol | | | | | | 0.05 |
| | Salicylic acid | | | | | | 0.01 |
| | Water | Balance | Balance | Balance | Balance | Balance | Balance |
| Sum of effective coefficients | | 2.0 | 2.0 | 2.5 | 2.3 | 4.5 | 2.8 |
| Evaluation | Residual ratio (40°C 1W) | 80 | 76 | 72 | 88 | 42 | 47 |
| | Evaluation of stability over time | A | A | A | A | C | C |
| | Evaluation of antiseptic performance | A | A | A | A | C fungi | A |

EP 2 818 157 A1

13

[Table 2]

| Composition | | Example 5 | Example 6 | Example 7 | Comparative Example 3 | Comparative Example 4 | Comparative Example 5 | Reference Example 1 |
|---|---|---|---|---|---|---|---|---|
| Composition | Tomato extract | 0.15 | 0.15 | 0.15 | 0.15 | 0.15 | 0.15 | - |
| | Krill extract | - | - | - | - | - | - | -0.15 |
| | Squalane | 8.00 | 8.00 | 8.00 | 8.00 | 8.00 | 8.00 | 8.00 |
| | Behenyl alcohol | 2.00 | 2.00 | 2.00 | 2.00 | 2.00 | 2.00 | 2.00 |
| | Cetearyl alcohol | 2.00 | 2.00 | 2.00 | 2.00 | 2.00 | 2.00 | 2.00 |
| | Glyceryl stearate | 4.00 | 4.00 | 4.00 | 4.00 | 4.00 | 4.00 | 4.00 |
| | Lecithin | 0.50 | 0.50 | 0.50 | 0.50 | 0.50 | 0.50 | 0.50 |
| | Decaglyceryl oleate | 0.50 | 0.50 | 0.50 | 0.50 | 0.50 | 0.50 | 0.50 |
| | Sucrose stearic acid ester | 0.50 | 0.50 | 0.50 | 0.50 | 0.50 | 0.50 | 0.50 |
| | Pentylene glycol | 1.50 | 1.50 | 1.50 | 1.50 | 1.50 | 1.50 | 1.50 |
| | Water | Balance | Balance | Balance | Balance | Balance | Balance | Balance |
| | Iodopropynyl butylcarbamate | 0.014 | 0.006 | 0.001 | | | | |
| | Phenoxyethanol | | 0.30 | | | | | |
| | Ethylhexylglycerin | | | 0.30 | | | | |
| | Methylparaben | | | | | 0.20 | 0.15 | 0.15 |
| | Ethylparaben | | | | | 0.10 | 0.10 | 0.10 |
| Sum of effective coefficients | | 1.97 | 1.92 | 2.17 | 0.57 | 2.57 | 2.32 | 2.32 |
| Evaluation | Residual ratio (40°C 4W) | 91 | 86 | 88 | 89 | 65 | 69 | 93 |
| | Evaluation of stability over time | A | A | A | A | B | B | A |
| | Evaluation of antiseptic performance | A | A | A | C | A | A | A |

EP 2 818 157 A1

14

[Examples 8 to 14]

<Production of Cosmetic Preparations>

Cosmetic preparations having the compositions indicated in the following Table 3 were produced by conventional methods, using the lycopene-containing emulsified composition obtained as described above, and ascorbic acid. The numerical values explaining the composition in Table 3 represent "mass%".

In Table 3, for ascorbic acid, a product manufactured by Wako Pure Chemical Industries, Ltd. was used.

<Evaluation>

The stability over time of the cosmetic preparations obtained as described above was evaluated by measuring the residual ratio of lycopene. Also, evaluations on antiseptic performance and odor were carried out simultaneously. The evaluation methods for stability over time and antiseptic performance were the same as the evaluation methods for stability over time and antiseptic performance carried out in Example 1.

<Odor>

Ascorbic acid undergoes oxidative decomposition over time, and generates a caramel-like odor (a burnt sweet smell). Therefore, a cosmetic product containing ascorbic acid tends to have the product value deteriorated due to changes over time.
Five subjects actually smelled the odors of the cosmetic preparations indicated in Table 3, which contained ascorbic acid, and evaluated the cosmetic preparations on the change in odor over time according to the following criteria.
OK: When all the subjects considered that the preparation was at a level without any problem with the product value
NG: When even only one of the subjects felt a caramel-like odor

[Table 3]

| | | Example 8 | Example 9 | Example 10 | Example 11 | Example 12 | Example 13 | Example 14 |
|---|---|---|---|---|---|---|---|---|
| Composition | Lycopene-containing emulsified composition | 0.20 | 0.20 | 0.20 | 0.20 | 0.20 | 0.20 | 0.20 |
| | Magnesium ascorbate phosphate | 0.10 | 0.10 | 0.10 | 0.10 | 0.10 | 0.10 | 0.10 |
| | Propylene glycol | 2.00 | 2.00 | 2.00 | 2.00 | 2.00 | 2.00 | 2.00 |
| | Pentylene glycol | 2.00 | 2.00 | 2.00 | 2.00 | 2.00 | 2.00 | 2.00 |
| | Water | Balance | Balance | Balance | Balance | Balance | Balance | Balance |
| | Iodopropynyl butylcarbamate | 0.002 | 0.002 | 0.002 | 0.002 | 0.002 | 0.008 | 0.001 |
| | Phenoxyethanol | 0.25 | 0.25 | 0.25 | 0.25 | 0.25 | - | - |
| | Ethylhexylglycerin | - | - | - | - | - | - | 0.25 |
| | Ascrobic acid | 0.01 | 0.10 | 0.30 | 0.50 | 1.00 | 0.30 | 0.30 |
| Evaluation | Residual ratio (40°C 2W) | 90 | 93 | 96 | 99 | 100 | 97 | 96 |
| | Evaluation | A | A | A | A | A | A | A |
| | Antiseptic performance (Challenge test) | A | A | A | A | A | A | A |
| | Odor | OK | OK | OK | OK | NG | OK | OK |

EP 2 818 157 A1

As can be seen from Table 3, it was found that when ascorbic acid was added, the residual ratio of lycopene was restored to a large extent. Also, when ascorbic acid was added in an amount of 1.0% by mass or more, an odor that would cause a problem in the product value occurred. Therefore, it was made clear that a very stable lycopene-containing composition is obtained by suppressing the amount of addition of ascorbic acid to less than 1.0% by mass. This was an unexpected result, and it is contemplated that this is an effect that cannot be easily conceived by those ordinarily skilled in the art.

It was confirmed that even in cosmetic preparations produced using a tomato extract instead of a lycopene emulsion, the residual ratio of lycopene was similarly restored to a large extent by adding ascorbic acid.

[Example 15 to Example 21]

In the following, external preparations for skin were produced according to various formulations, respectively by conventional methods. The numerical values shown below mean mass percentage (mass%) with respect to the total mass (100%) of each formulation.

The lycopene-containing emulsified composition and the astaxanthin-containing emulsified composition were compositions produced as described above.

In the following, the tomato extract and the Haematococcus algae extract were the same as the extracts used in Examples 1 to 7.

"GLYCACIL 2000" (manufactured by Lonza Group AG) contained 6% by mass of iodopropynyl butylcarbamate.

Example 15: Emulsion

| (Component) | (mass%) |
|---|---|
| • Oil phase component | |
| Tomato extract | 0.1 |
| Lycopene-containing emulsified composition | 0.1 |
| Haematococcus algae extract | 0.5 |
| Astaxanthin-containing emulsified composition | 0.5 |
| Squalane | 8.0 |
| Jojoba oil | 7.0 |
| Cetyl alcohol | 1.5 |
| • Aqueous phase component | |
| Glycerin monostearate | 2.0 |
| Polyoxyethylene cetyl ether | 3.0 |
| Polyoxyethylene sorbitan monooleate | 2.0 |
| 1,3-Butylene glycol | 1.0 |
| Glycerin | 2.0 |
| GLYCACIL 2000 | 0.2 |
| Sucrose stearate | 0.1 |
| Polyglyceryl-10 oleate | 0.1 |
| Polyglyceryl-2 stearate | 0.1 |
| Phenoxyethanol | 0.2 |
| Collagen | 1.0 |
| Sodium citrate | 1.0 |
| Fragrance | Adequate amount |
| Purified water | Balance |
| Sum of effective coefficient I and total of effective coefficients II: | 1.8 |

Example 16: Cream

| (Component) | (mass%) |
|---|---|
| Tomato extract | 0.2 |
| Krill extract | 0.3 |
| Cetostearyl alcohol | 3.0 |
| Glycerin fatty acid ester | 2.0 |
| Polyoxyethylene(20) sorbitan monooleate | 1.0 |
| Sorbitan monostearate | 1.0 |
| N-stearoyl-N-methyltaurine sodium | 0.5 |
| Petrolatum | 5.0 |
| Lecithin | 0.5 |
| Pentylene glycol | 2.0 |
| Phenoxyethanol | 0.2 |
| Tocopherol | 0.01 |
| 1,3-BG | 1.0 |
| Dimethylpolysiloxane (100 mPa·s) | 3.0 |
| Glyceryl tri-2-ethylhexanoate | 20.0 |
| Lactic acid | 1.0 |
| Magnesium ascorbate phosphate | 0.5 |
| Dipropylene glycol | 10.0 |
| Sodium citrate | 0.5 |
| Titanium oxide | 0.1 |
| Fragrance | Adequate amount |
| Disodium edetate | 0.03 |
| Iodopropynyl butylcarbamate | 0.01 |
| Purified water | Balance |
| Sum of effective coefficient I and total of effective coefficients II: | 2.35 |

Example 17: Jelly-like essence

| (Component) | (mass%) |
|---|---|
| Tomato extract | 0.05 |
| Lycopene-containing emulsified composition | 0.05 |
| Haematococcus algae extract | 0.1 |
| Astaxanthin-containing emulsified composition | 0.2 |
| Mixture of ceramides III and VI | 1.0 |
| Hydrolyzed collagen | 1.0 |
| GLYCACIL 2000 | 0.1 |
| Acetylhydroxyproline | 1.0 |
| Ethylhexylglycerin | 0.1 |
| (PEG-20/decyltetradeceth 20/HDI) copolymer | 0.3 |
| Oleic acid | 0.5 |
| 1,3-Butylene glycol | 1.0 |
| Glycerin | 2.0 |
| Sucrose | 0.1 |
| Polyglyceryl-10 oleate | 0.1 |
| Polyglyceryl-2 stearate | 0.1 |
| Phenoxyethanol | 0.2 |
| Collagen | 1.0 |
| Sodium citrate | 1.0 |
| Sodium ascorbate | 0.05 |

(continued)

| (Component) | (mass%) |
| --- | --- |
| Damask rose oil | Adequate amount |
| Fragrance | Adequate amount |
| Purified water | Balance |
| Sum of effective coefficient I and total of effective coefficients II: 2.45 | |

Example 18: Sunscreen preparation

| (Component) | (mass%) |
| --- | --- |
| Tomato extract | 0.1 |
| Lycopene-containing emulsified composition | 0.1 |
| Haematococcus algae extract | 0.1 |
| Astaxanthin-containing emulsified composition | 0.1 |
| Squalane | 3.0 |
| Cyclopentasiloxane | 3.0 |
| Titanium oxide | 1.5 |
| AL hydroxide | 1.5 |
| t-butylmethoxydibenzoylmethane | 1.0 |
| Ethylhexyl methoxysilicate | 2.0 |
| PEG-30 dipolyhydroxystearate | 0.5 |
| Xanthan gum | 0.05 |
| Mica | 0.2 |
| Iron oxide | 0.01 |
| 1,3-Butylene glycol | 1.0 |
| Pentylene glycol | 2.0 |
| Glycerin | 2.0 |
| GLYCACIL 2000 | 0.15 |
| Sucrose stearate | 0.1 |
| Lecithin | 0.1 |
| Silica | 0.1 |
| Polyglyceryl-10 oleate | 0.1 |
| Polyglyceryl-2 stearate | 0.1 |
| Phenoxyethanol | 0.2 |
| Hydrolyzed collagen | 1.0 |
| Sodium hyaluronate | 0.5 |
| Fragrance | Adequate amount |
| Purified water | Balance |
| Sum of effective coefficient I and total of effective coefficients II: 2.25 | |

Example 19: Cosmetic product for pack

| (Component) | (mass%) |
| --- | --- |
| Lycopene-containing emulsified composition | 0.1 |
| Haematococcus algae extract | 0.1 |
| Butylene glycol | 5.0 |
| Glycerin | 10.0 |
| Glyceryl stearate | 0.3 |

(continued)

| (Component) | (mass%) |
|---|---|
| Pentylene glycol | 1.0 |
| Isotridecyl isononanoate | 1.0 |
| Phenoxyethanol | 0.5 |
| CARBOMER | 0.3 |
| Acetylhydroxyproline | 0.1 |
| Hydrolyzed collagen | 0.1 |
| Ascorbic acid palmitate | 0.5 |
| Magnesium ascorbate phosphate | 0.2 |
| GLYCACIL 2000 | 0.1 |
| Ethylhexylglycerin | 0.1 |
| Methylparaben | 0.05 |
| Purified water | Balance |
| Sum of effective coefficient I and total of effective coefficients II: 2.48 | |

Example 20: Emulsion

| (Component) | (mass%) |
|---|---|
| • Oil phase component | |
| Tomato extract | 0.1 |
| Lycopene-containing emulsified composition | 0.1 |
| Haematococcus algae extract | 0.5 |
| Astaxanthin-containing emulsified composition | 0.5 |
| Squalane | 8.0 |
| Jojoba oil | 7.0 |
| Cetyl alcohol | 1.5 |
| • Aqueous phase component | |
| Glycerin monostearate | 2.0 |
| Polyoxyethylene cetyl ether | 3.0 |
| Polyoxyethylene sorbitan monooleate | 2.0 |
| 1,3-Butylene glycol | 1.0 |
| Glycerin | 2.0 |
| GLYCACIL 2000 | 0.2 |
| Sucrose stearate | 0.1 |
| Polyglyceryl-10 oleate | 0.1 |
| Polyglyceryl-2 stearate | 0.1 |
| Phenoxyethanol | 0.2 |
| Collagen | 1.0 |
| Sodium citrate | 1.0 |
| Ascorbic acid | 0.02 |
| Fragrance | Adequate amount |
| Purified water | Balance |
| Sum of effective coefficient I and total of effective coefficient II: 1.8 | |

Example 21: Cream

| (Component) | (mass%) |
| --- | --- |
| Tomato extract | 0.2 |
| Krill extract | 0.3 |
| Cetostearyl alcohol | 3.0 |
| Glycerin fatty acid ester | 3.0 |
| Polyoxyethylene(20) sorbitan monooleate | 1.0 |
| Sorbitan monostearate | 1.0 |
| N-stearoyl-N-methyltaurine sodium | 0.5 |
| Petrolatum | 6.0 |
| Lecithin | 0.5 |
| Pentylene glycol | 2.0 |
| Phenoxyethanol | 0.2 |
| Tocopherol | 0.05 |
| 1,3-BG | 1.0 |
| Dimethylpolysiloxane (100 mPa·s) | 3.0 |
| Glyceryl tri-2-ethylhexanoate | 22.0 |
| Lactic acid | 1.0 |
| Magnesium ascorbate phosphate | 0.1 |
| Dipropylene glycol | 10.0 |
| Sodium citrate | 0.5 |
| Ascorbic acid | 0.4 |
| Titanium oxide | 0.1 |
| Fragrance | Adequate amount |
| Disodium edetate | 0.03 |
| Iodopropynyl butylcarbamate | 0.01 |
| Purified water | Balance |
| Sum of effective coefficient I and total of effective coefficients II: 2.35 | |

Example 22: Skin toner

| (Component) | (mass%) |
| --- | --- |
| Lycopene-containing emulsified composition | 0.1 |
| Astaxanthin-containing emulsified composition | 0.5 |
| Pentylene glycol | 2.0 |
| 1,3-Butylene glycol | 1.5 |
| Glycerin | 1.0 |
| Propylene glycol | 2.0 |
| GLYCACIL 2000 | 0.2 |
| Phenoxyethanol | 0.2 |
| Hydrolyzed collagen | 0.5 |
| Water-soluble collagen | 0.5 |
| Acetylhydroxyproline | 0.5 |
| Arginine | 0.1 |
| Xanthan gum | 0.05 |
| Royal jelly extract | 0.1 |
| PULLULAN | 0.1 |
| Glucosyl rutin | 0.1 |
| Glucosyl trehalose | 0.1 |

(continued)

| (Component) | (mass%) |
|---|---|
| Betaine | 1.0 |
| *Pterocarpus marsupium* bark extract | 0.1 |
| Sorbitol | 0.1 |
| Sodium citrate | 1.0 |
| Polyoxyethylene hardened castor oil | 0.2 |
| Sodium ascorbate | 0.3 |
| Magnesium ascorbate phosphate | 0.5 |
| Sodium hyaluronate | 0.2 |
| Damask rose oil | Adequate amount |
| Fragrance | Adequate amount |
| Purified water | Balance |
| Sum of effective coefficient I and total of effective coefficients II: 2.8 | |

According to the invention, a lycopene-containing composition which has sufficient antiseptic performance and can have lycopene stably incorporated therein, can be provided.

The entire disclosures of Japanese Patent Application Nos. 2012-039372 and 2012-213109 are incorporated herein by reference.

All publications, patent applications, and technical standards mentioned in this specification are herein incorporated by reference to the same extent as if each individual publication, patent application, or technical standard was specifically and individually indicated to be incorporated by reference.


**Claims**

1.   A lycopene-containing composition comprising lycopene and iodopropynyl butylcarbamate.

2.   The lycopene-containing composition according to claim 1, wherein:

   the composition further comprises at least one antibiotic compound having a minimum inhibitory concentration (MIC) against *Escherichia coli* of 10% by mass or less, and
   the antibiotic compound includes one selected from the group consisting of phenoxyethanol, an ether compound having from 6 to 11 carbon atoms in total formed between glycerin and an aliphatic alcohol, and an aliphatic diol compound having a carbon chain length of from 3 to 10.

3.   The lycopene-containing composition according to claim 2, wherein a sum of an effective coefficient of iodopropynyl butylcarbamate represented by the following Formula (I), and a total of effective coefficients of the antibiotic compound represented by the following Formula (II) is from 1.5 to 4.5:

$$\text{Effective coefficient I} = [\text{Content (mass\%) of iodopropynyl butylcarbamate}] \, / \, [\text{minimum inhibitory concentration (MIC) (mass\%) of iodopropynyl butylcarbamate against } \textit{Escherichia coli}] \quad (I)$$

$$\text{Effective coefficient II} = [\text{Content (mass\%) of antibiotic compound}] \, / \, [\text{minimum inhibitory concentration (MIC) (mass\%) of antibiotic compound against } \textit{Escherichia coli}] \quad (II).$$

4.   The lycopene-containing composition according to claim 2 or 3, wherein the ether compound formed between glycerin and an aliphatic alcohol is ethylhexylglycerin.

5. The lycopene-containing composition according to any one of claims 2 to 4, wherein the aliphatic diol compound is at least one selected from pentylene glycol, hexylene glycol, heptanediol, or octanediol.

6. The lycopene-containing composition according to any one of claims 1 to 5, further comprising ascorbic acid.

7. The lycopene-containing composition according to claim 6, wherein a content of the ascorbic acid is from 0.01% by mass to 0.5% by mass with respect to a total mass of the composition.

8. The lycopene-containing composition according to any one of claims 1 to 7, wherein a content of the iodopropynyl butylcarbamate is from 0.0001% by mass to 0.5% by mass with respect to a total mass of the composition.

9. The lycopene-containing composition according to any one of claims 1 to 9, wherein the lycopene-containing composition is an oil-in-water type emulsified composition comprising lycopene as one of oil phase components, and a content ratio of aqueous phase components is 70% by mass or more with respect to a total amount of the composition.

10. An external preparation for skin, comprising the lycopene-containing composition according to any one of claims 1 to 9.

## INTERNATIONAL SEARCH REPORT

International application No.

PCT/JP2013/051213

A. CLASSIFICATION OF SUBJECT MATTER
*A61K8/42*(2006.01)i, *A61K8/31*(2006.01)i, *A61Q19/00*(2006.01)i

According to International Patent Classification (IPC) or to both national classification and IPC

B. FIELDS SEARCHED

Minimum documentation searched (classification system followed by classification symbols)
A61K8/42, A61K8/31, A61Q19/00

Documentation searched other than minimum documentation to the extent that such documents are included in the fields searched
| | | | |
|---|---|---|---|
| Jitsuyo Shinan Koho | 1922–1996 | Jitsuyo Shinan Toroku Koho | 1996–2013 |
| Kokai Jitsuyo Shinan Koho | 1971–2013 | Toroku Jitsuyo Shinan Koho | 1994–2013 |

Electronic data base consulted during the international search (name of data base and, where practicable, search terms used)
CA/REGISTRY(STN), JSTPlus(JDreamII), JMEDPlus(JDreamII), JST7580(JDreamII)

C. DOCUMENTS CONSIDERED TO BE RELEVANT

| Category* | Citation of document, with indication, where appropriate, of the relevant passages | Relevant to claim No. |
|---|---|---|
| X<br>Y | WO 2006/069426 A2 (NATURA COSMÉTICOS S.A.),<br>06 July 2006 (06.07.2006),<br>page 6, line 32 to page 7, line 22; page 9,<br>line 17 to page 10, line 3; page 12, lines 23<br>to 29; page 16, lines 5 to 24; example 3<br>& US 2009/0023628 A1 & EP 1830794 A2<br>& CA 2591764 A | 1-3,6-10<br>4,5 |
| Y | JP 2010-229081 A (Mandom Corp.),<br>14 October 2010 (14.10.2010),<br>claim 4; paragraph [0027]; blending examples 1,<br>2<br>(Family: none) | 4,5 |

☒ Further documents are listed in the continuation of Box C. ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 15 February, 2013 (15.02.13) | 26 February, 2013 (26.02.13) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.

PCT/JP2013/051213

C (Continuation).    DOCUMENTS CONSIDERED TO BE RELEVANT

| Category* | Citation of document, with indication, where appropriate, of the relevant passages | Relevant to claim No. |
|---|---|---|
| Y | JP 2008-120712 A  (Q.P. Corp.),<br>29 May 2008 (29.05.2008),<br>claims 1, 5, 6<br>(Family: none) | 4,5 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002125619 A
- JP H1135444 A
- JP 4398246 B
- JP 3921305 B
- JP H05148127 A
- JP 2010229081 A
- JP 2012039372 A
- JP 2012213109 A

**Non-patent literature cited in the description**

- *Japanese Pharmacopeia,* 2006
- 28. Preservatives-Effectiveness Tests. *Japanese Pharmacopoeia,* 2006